# EUROPEAN PATENT APPLICATION

(11) **EP 3 594 666 A1**
(43) Date of publication of application: **15.01.2020**
(21) Application number: 18197544.2
(22) Date of filing: 28.09.2018
(51) Int. Cl.: G01N 21/88, G01N 33/44, G01N 21/95, C14B 17/00, C14B 1/28

(54) **ARTIFICIAL INTELLIGENCE-BASED LEATHER INSPECTION METHOD AND LEATHER PRODUCT PRODUCTION METHOD**

(30) Priority: 12.07.2018 TW 107124164; 18.09.2018 TW 107132719
(71) Applicant: Aibi Dynamics Co., Ltd., 407 Taichung City (TW)
(72) Inventor: CHANG, Yu-Pin, 407 TAICHUNG CITY (TW)
(74) Representative: Becker Kurig Straus

(57) **Abstract**

An artificial intelligence-based leather inspection method and leather product pr0oduction method includes the step of using sensor means (16) to obtain a leather data of a leather raw material (10), then the step of inputting the leather data to an artificial intelligence module (20) to determine a defective area (22) and a non-defective area (24) of the leather raw material (10), the step of establishing an area data after judgment of the defective area (22) and the non-defective area (24) and then using the area data to define the non-defective area (24) into one or multiple reserved areas (32) so that the leather raw material (10) can be cut into leather components (50) corresponding to the respective reserved areas (32).

## Description

### BACKGROUND OF THE INVENTION

### 1. Field of the Invention

The present invention relates to leather technology and more particularly, to an artificial intelligence-based leather inspection method and leather product production method.

### 2. Description of the Related Art

Leather can be used in a wide range of consumer goods, such as clothing, purses, luggage or decorative accessories, etc., which are everyday items that are often used. Because natural leather (also known as genuine leather) has good touch and high durability, high-priced and high-value products often use natural leather as the main material.

Natural leather is susceptible to damage from raw materials or manufacturing processes, such as damage and defects on the leather surface or internal tissues due to animal injury, mildew, pests, ruptures, or transport bumping. In order to allow the leather products to be inspected beforehand, most of the time, when the leather is still in the raw material state, the leather is inspected in detail by visual inspection or manual inspection, and then the leather surface is marked to indicate the defective areas. The inspected and marked leathers are then properly cut and sorted into the available leather parts for subsequent production according to the required characteristics of the finished product.

However, the above-mentioned inspection method using human visual inspection or hand inspection is not only time consuming, it needs to employ an inspector with sufficient experience to judge the defect, and the training and cultivation process of the inspection personnel is long and difficult. Because the judgment method relies on more subjective visual or sensory inspection, it is difficult to establish more consistent and universal quality standards due to factors such as personal emotion, environment or time and space.

### SUMMARY OF THE INVENTION

The present invention has been accomplished under the circumstances in view. It is the main object of the present invention to provide an artificial intelligence-based leather inspection method and leather product production method, which can greatly reduce the leather inspection time, establish a consistent and universal leather quality inspection standard, and at the same time realize the automated production process of leather products and improve the overall production efficiency.

To achieve this and other objects of the present invention, an artificial intelligence-based leather inspection method includes the step of using sensor means to obtain a leather data of a leather raw material, and then the step of inputting the leather data to an artificial intelligence module to determine a defective area and a non-defective area of the leather raw material.

Preferably, the method is to obtain local leather data of the leather raw material at different locations, and then to integrate all the local leather data into the leather data of the leather raw material.

Preferably, the artificial intelligence-based leather inspection method further comprising a sub step of establishing an area data after judgment of the defective area and the non-defective area, and then using the area data to define the non-defective area into at least one reserved area.

Preferably, the artificial intelligence-based leather inspection method further comprising a sub step of projecting a light source onto said leather raw material, wherein the lighting characteristics of the light source is adjustable according to the material characteristics of the leather raw material.

Preferably, the artificial intelligence module comprises a deep learning model.

Other advantages and features of the present invention will be fully understood by reference to the following specification in conjunction with the accompanying drawings, in which like reference signs denote like components of structure.

### BRIEF DESCRIPTION OF THE DRAWINGS

FIG. 1 is a system architecture block diagram of the present invention.
FIG. 2 is a schematic drawing of the present invention, illustrating one implementation example of the leather data collection device.
FIG. 3 is a schematic drawing of the present invention, illustrating another implementation example of the leather data collection device.
FIG. 4 is a schematic drawing illustrating the configuration of the leather inspection platform.
FIG. 5 corresponds to FIG. 4, illustrating a leather raw material placed on the leather inspection platform.
FIG. 6 is a schematic drawing illustrating detective areas of the leather raw material.
FIG. 7 is a schematic drawing illustrating the non-detective areas of the leather raw material after typesetting.
FIG. 8 is a schematic drawing illustrating the leather raw material cut into leather components.

### DETAILED DESCRIPTION OF THE INVENTION

Prior to viewing the following specification in conjunction with the accompanying drawings, it is to be understood that the artificial intelligence-based leather inspection method and leather product production method of the present invention can be widely used to inspect various types or surface treatments of natural leather or synthetic leather. Those skilled in the art should be able to understand that the artificial intelligence, the operating instructions, and the operating steps in the present preferred embodiment are all superordinated descriptions that do not limit the specific calculus model, the technical field, or the operation sequence. And for the quantitative term "a" is meant to include one and more than a plurality of components

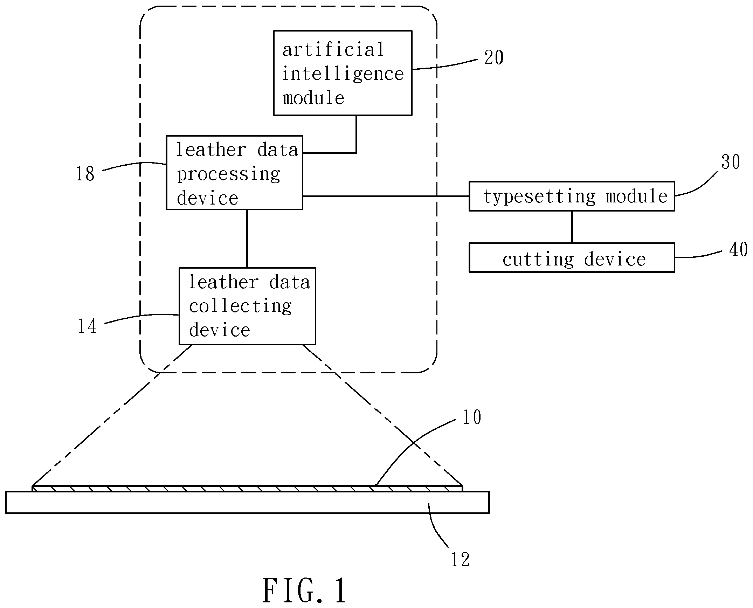

Referring to FIGS. 1-4, an artificial intelligence-based leather inspection method comprises the steps of (A) data collection, (B) data processing, and (C) generation of process data.

In Step (A) of data collection, place a leather raw material **10** on a leather inspection platform **12,** and then use a leather data collecting device **14** in the leather inspection platform **12** to obtain the leather data of the leather raw material **10.** In the present preferred embodiment, the leather raw material **10** is a natural leather, however, this step is also applicable to other kinds of leathers. In the present preferred embodiment, the leather data collecting device **14** is an optical sensor device adapted for photographing the surface of the leather raw material **10** to obtain a digital image of the leather surface so as to further form leather data for judging the edge and surface state of the leather raw material **10.**

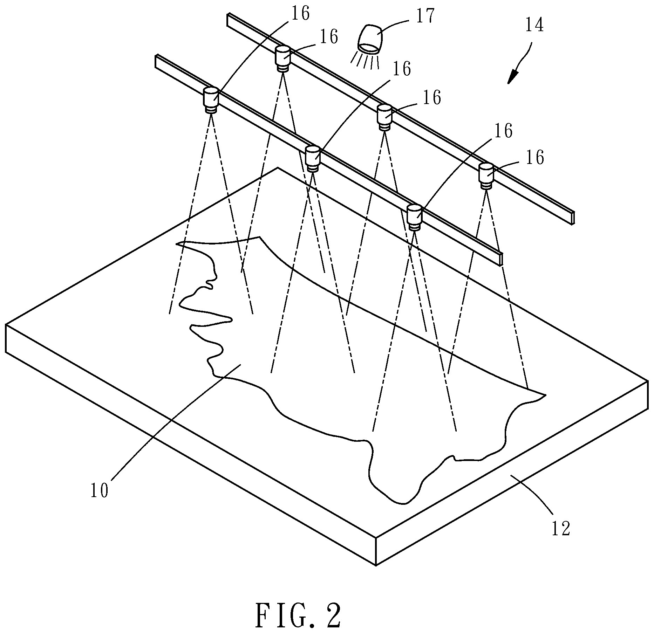

As shown in FIG. 2, the leather data collecting device **14** comprises a plurality of sensor components **16** that are arranged in an array and evenly distributed over the leather raw material **10.** Each of the sensor components **16** obtains local leather data of the leather raw material **10** at different locations. The leather data collecting device **14** further comprises a light source **17** adapted to emit light onto the leather raw material **10** for enabling the sensor components **16** to obtain complete digital leather data. The light source **17** can be a point light source or an array light source. In order to cooperate with the material types, different material treatments or different leather raw material surface textures, the light source **17** can project different illumination characteristics according to the material characteristics of different leather raw materials **10,** that is, the intensity, illuminance, or brightness of the light source can be adjusted with the leather raw material **10** to be inspected.

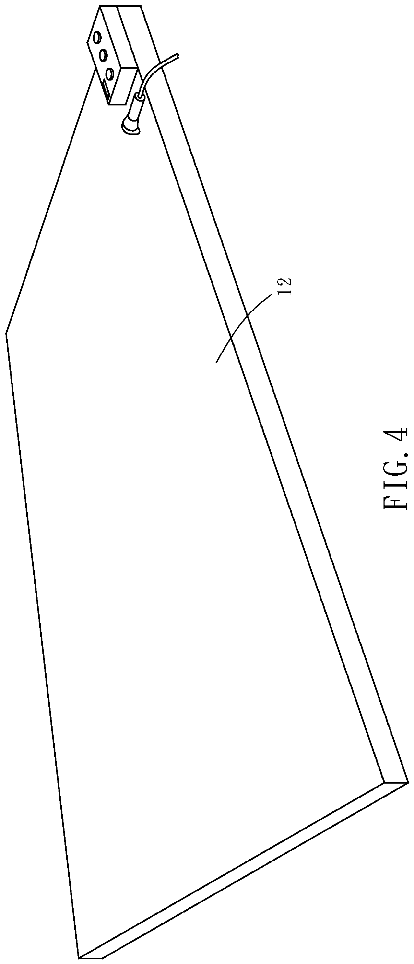

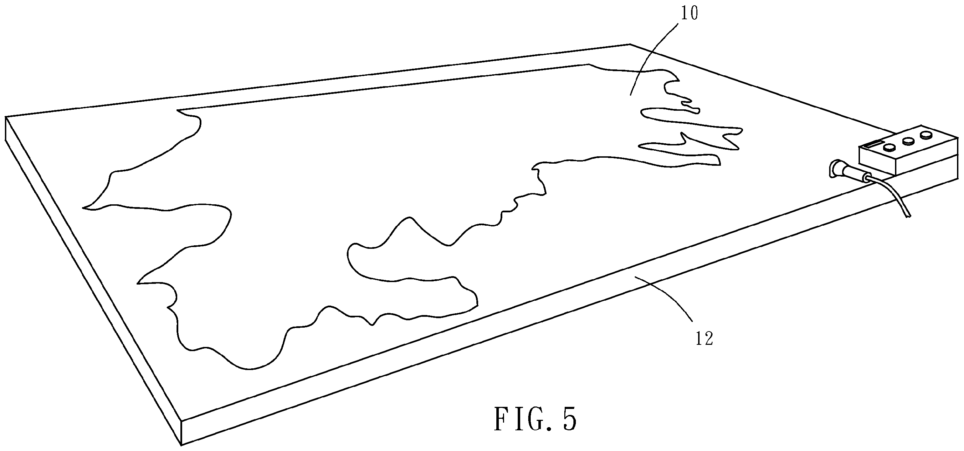

In Step (B) of data processing, as shown in FIG. 4 and FIG. 5, after the leather raw material **10** is placed flat on the leather inspection platform **12** and the leather data is obtained from the leather data collecting device **14** above the leather inspection platform **12,** input the leather data into a leather data processing device **18** for calculation procedures. In the present preferred embodiment, the leather data processing device **18** comprises at least one image processing module for splicing and integrating the local leather data obtained by the leather data collecting device **14** into complete leather data.

The leather data processing device **18** further comprises an artificial intelligence module **20.** The artificial intelligence module **20** of the present preferred embodiment is an example including a deep learning model for calculating and judging the defective area **22** and the non-defective area **24** of the surface of the leather raw material **10.**

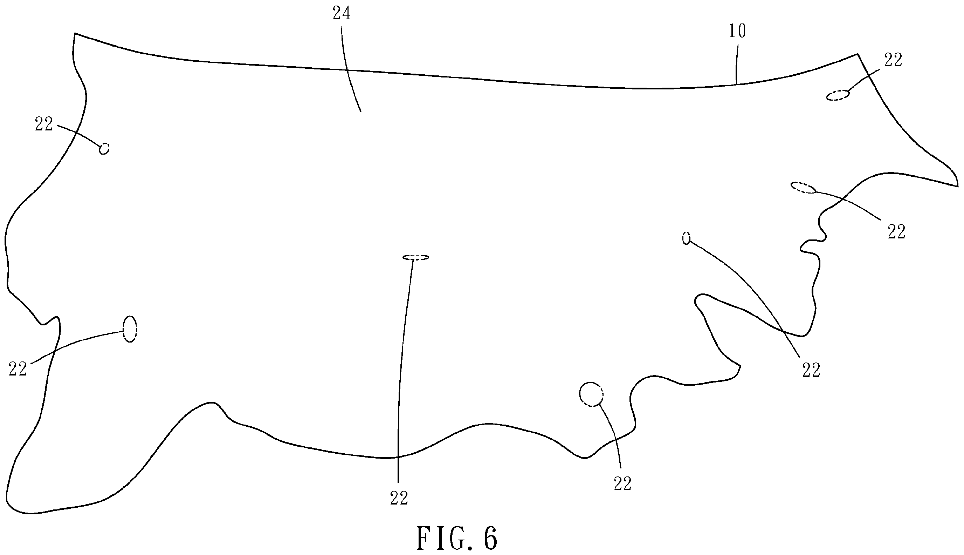

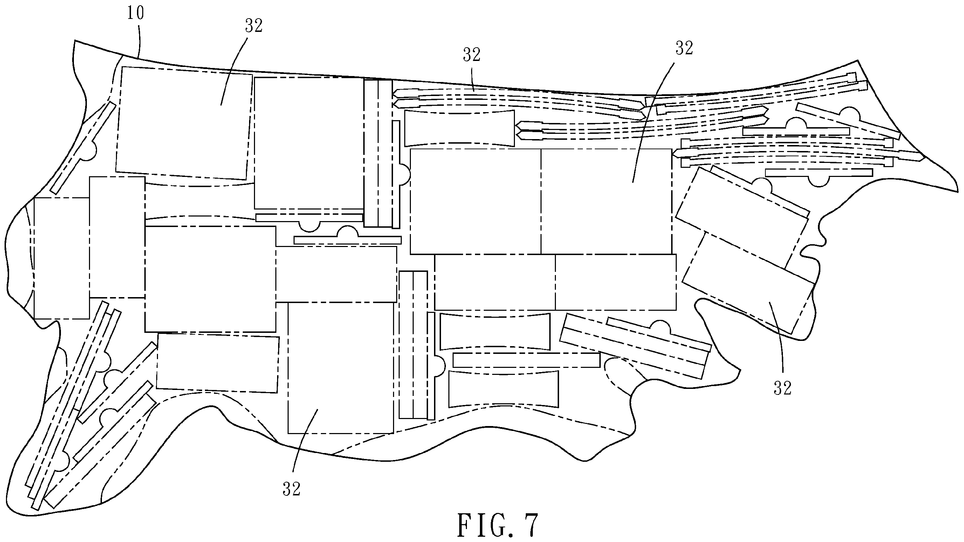

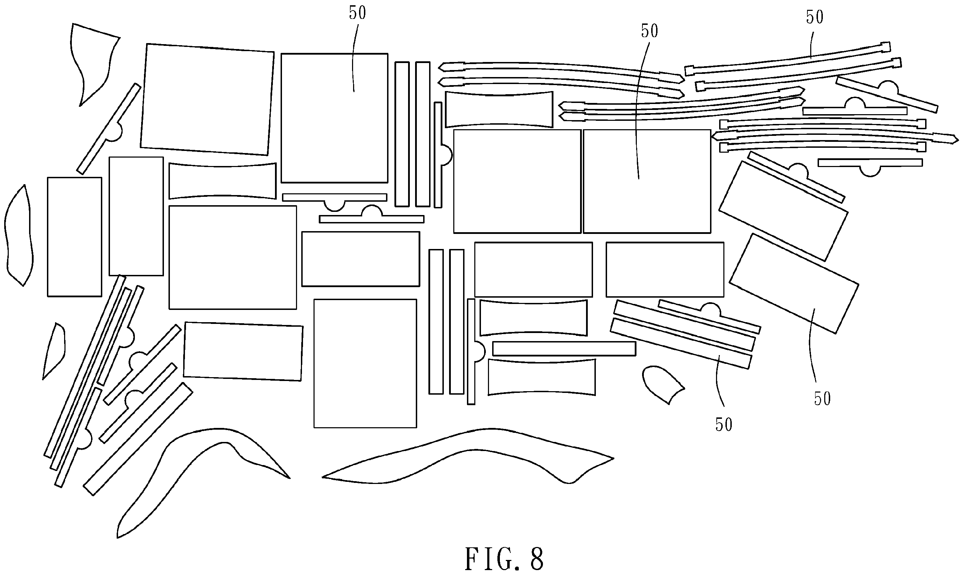

In step (C) of generation of process data, as shown in FIG. 1 and FIGS. 6-8, use a typesetting module **30** to establish the area data of the leather raw material **10** after judgment of the defective area **22** and the non-defective area **24** of the surface of the leather raw material **10** by the artificial intelligence module **20** of the leather data processing device **18,** and then use the area data to define the non-defective area **24** into at least one reserved area **32.** In the subsequent leather product production method, the leather raw material **10** is cut by a cutting device **40** to produce at least one leather component **50** corresponding to the at least one reserved area **32.**

Based on the aforesaid artificial intelligence-based leather inspection method and leather product production method, the present invention has at least the following technical effects:
1. Using the artificial intelligence module with deep learning model, you can judge the defects of leather without manual, greatly reducing leather inspection time.
2. The invention can quickly complete the inspection without considering the inspection environment, time or human factors, and establish a consistent and universal leather quality inspection standard.
3. The leather inspection method is combined with the subsequent typesetting and cutting process to improve the utilization of the raw material is improved.
4. The invention can integrate the leather raw material quality inspection into the subsequent cutting process to realize the automatic production process of the leather product.

It is worth mentioning that the above-mentioned leather data collecting device can also obtain the internal organization or material state of the leather raw material by using a device to produce a collapse effect on leather raw material by means of transmission or mechanical force. For example, use an X-ray device to irradiate X-rays to the leather raw material, and then analyze the X-ray signal thus obtained to find out the characteristics of the inner organization of the leather raw material.

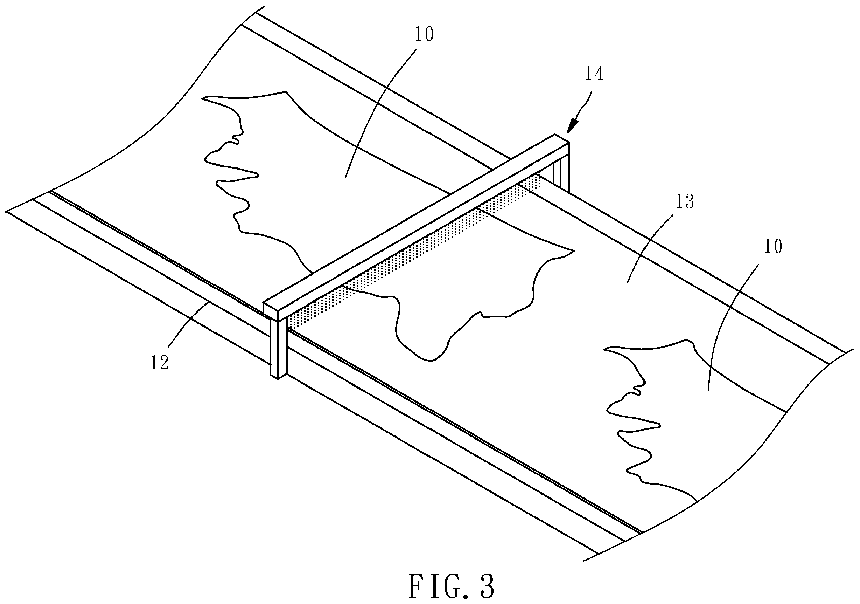

As shown in FIG. 3, the leather data collecting device **14** can scan the leather surface of the leather raw material **10** to obtain the desired leather data when the leather raw material **10** is moving along with a conveyor belt **13** over the leather inspection platform **12,** thereby improving the overall inspection and production efficiency.

Further, in addition to the deep learning model, the artificial intelligence module can use other machine learning models such as neural network models, convolutional network models, or cyclic neural network models to enhance the accuracy and precision of artificial intelligence.

## Claims

1. An artificial intelligence-based leather inspection method, which is **characterized in that** said method comprises the steps of:
(a): using sensor means (16) to obtain a leather data of a leather raw material (10); and
(b): inputting said leather data to an artificial intelligence module (20) to determine a defective area (22) and a non-defective area (24) of said leather raw material (10).

2. The artificial intelligence-based leather inspection method as claimed in claim 1, which is **characterized in that** said method comprises the step (a) is to obtain local leather data of said leather raw material (10) at different locations, and then to integrate all the local leather data into said leather data of said leather raw material (10).

3. The artificial intelligence-based leather inspection method as claimed in claim 1, which is **characterized in that** said method comprises a sub step of establishing an area data after judgment of said defective area (22) and said non-defective area (24), and then using said area data to define said non-defective area (24) into at least one reserved area (32).

4. The artificial intelligence-based leather inspection method as claimed in claim 1, which is **characterized in that** said artificial intelligence module (20) comprises a deep learning model.

5. The artificial intelligence-based leather inspection method as claimed in claim 1, which is **characterized in that** said sensor means (16) used in the step (a) is an image sensor for capturing an image of said leather raw material (10) to obtain said leather data.

6. The artificial intelligence-based leather inspection method as claimed in claim 5, which is **characterized in that** said sensor means (16) is used in the step (a) to obtain local leather data of said leather raw material (10) at different locations, and then to integrate all the local leather data into said leather data of said leather raw material (10).

7. The artificial intelligence-based leather inspection method as claimed in claim 5, which is **characterized in that** said sensor means (16) is used in the step (a) to obtain local leather data of said leather raw material by means of projecting a light source (17) onto said leather raw material (10), the lighting characteristics of said light source (17) being adjustable according to the material characteristics of said leather raw material (10).

8. A leather product production method, which is **characterized in that** said method comprises the steps of:
(a): employing the artificial intelligence-based leather inspection method as claimed in claim 1 to inspect a leather raw material (10); and
(b): cutting said leather raw material (10) to obtain leather components (50) corresponding to the defective area (22) and the non-defective area (24) of said leather raw material (10).

9. The leather product production method as claimed in claim 8, which is **characterized in that** said method comprises the step (b) of cutting said non-defective area (24) of said leather raw material (10) to obtain said leather components (50).
